# EUROPEAN PATENT SPECIFICATION

(11) **EP 3 099 812 B1**
(45) Date of publication and mention of the grant of the patent: **14.11.2018**
(21) Application number: 15701773.2
(22) Date of filing: 28.01.2015
(51) Int. Cl.: C12Q 1/6844, C12Q 1/686

(54) **METHOD AND KIT WITH INTERNAL AMPLIFICATION CONTROL**
METHODE UND KIT MIT INTERNE AMPLIFICATIONS-KONTROLLE
METHODE ET KIT AVEC CONTROLE INTERNE DE L'AMPLIFICATION

(30) Priority: 28.01.2014 EP 14152875
(43) Date of publication of application: 07.12.2016
(73) Proprietor: Qiagen GmbH, 40724 Hilden (DE)
(72) Inventor: STRAUSS, Sascha, 42697 Solingen (DE); MÜLLER, Daniel, 40724 Hilden (DE); SIEGMUND, Vanessa, 40227 Düsseldorf (DE)
(74) Representative: CH Kilger Anwaltspartnerschaft mbB
(86) International application number: PCT/EP2015/051710
(87) International publication number: WO 2015/114009

(56) References cited:
- WO-A1-2005/083114
- WO-A1-2012/113577
- WO-A2-2014/028531
- SWANGO K L ET AL: "A quantitative PCR assay for the assessment of DNA degradation in forensic samples", FORENSIC SCIENCE INTERNATIONAL, ELSEVIER SCIENTIFIC PUBLISHERS IRELAND LTD, IE, vol. 158, no. 1, 20 April 2006 (2006-04-20), pages 14-26, XP027939885, ISSN: 0379-0738 [retrieved on 2006-04-20]
- HUDLOW ET AL: "A quadruplex real-time qPCR assay for the simultaneous assessment of total human DNA, human male DNA, DNA degradation and the presence of PCR inhibitors in forensic samples: A diagnostic tool for STR typing", 20071126, vol. 2, no. 2, 26 November 2007 (2007-11-26), pages 108-125, XP022452115,
- VERDIN E ET AL: "Use of an internal control in a nested-PCR assay for Mycoplasma hyopneumoniae detection and quantification in tracheobronchiolar washings from pigs", MOLECULAR AND CELLULAR PROBES, ACADEMIC PRESS, LONDON, GB, vol. 14, no. 6, 1 November 2000 (2000-11-01), pages 365-372, XP004466403, ISSN: 0890-8508, DOI: 10.1006/MCPR.2000.0326
- D Bautista ET AL: "DNA Quantification by Gel Densitometry with Norgen DNA Ladders", , 1 January 2007 (2007-01-01), XP055125585, Retrieved from the Internet: URL:https://norgenbiotek.com/product_resou rces/minisizer_50_bp_dna_ladder_dna_quanti fication_by_gel_densitometry_with_norgen_d na_ladders_11200_307.pdf [retrieved on 2014-06-27] & Anonymous: "NORGEN BIOTEK CORPORATION-Catalogue 2007", , 1 January 2007 (2007-01-01), XP055125582, Retrieved from the Internet: URL:http://tecscience.com.br/novosite/wp-c ontent/uploads/2011/05/Catalogonorgenbiote k.pdf [retrieved on 2014-06-27]
- PIONZIO ARIANNA M ET AL: "The effect of internal control sequence and length on the response to PCR inhibition in real-time PCR quantitation", FORENSIC SCIENCE INTERNATIONAL: GENETICS, vol. 9, 31 October 2013 (2013-10-31), pages 55-60, XP028610825, ISSN: 1872-4973, DOI: 10.1016/J.FSIGEN.2013.10.010
- Anonymous: "AmpFlSTR Identifiler PCR amplification kit", , 1 August 2012 (2012-08-01), pages 1-141, XP055125647, Retrieved from the Internet: URL:http://www3.appliedbiosystems.com/cms/ groups/applied_markets_support/documents/g eneraldocuments/cms_041201.pdf [retrieved on 2014-06-27]
- NGUYEN V Q ET AL: "A large-scale validation of dosage analysis by robust dosage-polymerase chain reaction", ANALYTICAL BIOCHEMISTRY, ELSEVIER, AMSTERDAM, NL, vol. 371, no. 1, 1 December 2007 (2007-12-01), pages 37-42, XP026864790, ISSN: 0003-2697 [retrieved on 2007-10-10]

### SEQUENCE LISTING

<110> Qiagen GmbH
<120> Internal amplification control
<130> B051-0148WO1
<150> EP 14 15 2875.2
   <151> 2014-01-28
<160> 34
<170> PatentIn version 3.5
<210> 1
   <211> 24
   <212> DNA
   <213> Artificial Sequence
<220>
   <223> Primer
<400> 1
   tactccaacc acataatagt cctc 24
<210> 2
   <211> 24
   <212> DNA
   <213> Artificial sequence
<220>
   <223> Primer
<400> 2
   tctactccaa ccacataata gtcc 24
<210> 3
   <211> 24
   <212> DNA
   <213> Artificial sequence
<220>
   <223> Primer
<400> 3
   tcgctctact ccaaccacat aata 24
<210> 4
   <211> 21
   <212> DNA
   <213> Artificial sequence
<220>
   <223> Primer
<400> 4
   cagccgcgtg aggtctacaa a 21
<210> 5
   <211> 27
   <212> DNA
   <213> Artificial sequence
<220>
   <223> Primer
<400> 5
   attcgctcta ctccaaccac ataatag 27
<210> 6
   <211> 25
   <212> DNA
   <213> Artificial sequence
<220>
   <223> Primer
<400> 6
   gctccataat cagaagatgc gctac 25
<210> 7
   <211> 25
   <212> DNA
   <213> Artificial sequence
<220>
   <223> Primer
<400> 7
   gcgtagctcc ataatcagaa gatgc 25
<210> 8
   <211> 27
   <212> DNA
   <213> Artificial sequence
<220>
   <223> Primer
<400> 8
   gtaggcgtag ctccataatc agaagat 27
<210> 9
   <211> 24
   <212> DNA
   <213> Artificial sequence
<220>
   <223> Primer
<400> 9
   gtagctccat aatcagaaga tgcg 24
<210> 10
   <211> 24
   <212> DNA
   <213> Artificial sequence
<220>
   <223> Primer
<400> 10
   tggtggtgat tacgtatctt agtc 24
<210> 11
   <211> 24
   <212> DNA
   <213> Artificial sequence
<220>
   <223> Primer
<400> 11
   cctagcctaa gttcagatgt aagg 24
<210> 12
   <211> 24
   <212> DNA
   <213> Artificial sequence
<220>
   <223> Primer
<400> 12
   tcatttctgc tagtctacct tagc 24
<210> 13
   <211> 24
   <212> DNA
   <213> Artificial sequence
<220>
   <223> Primer
<400> 13
   tcatttctgc tagtctacct tagc 24
<210> 14
   <211> 22
   <212> DNA
   <213> Artificial sequence
<220>
   <223> Primer
<400> 14
   tcgtacgggc tatccactct ag 22
<210> 15
   <211> 24
   <212> DNA
   <213> Artificial sequence
<220>
   <223> Primer
<400> 15
   ctatccactc tagcaaacga ctta 24
<210> 16
   <211> 26
   <212> DNA
   <213> Artificial sequence
<220>
   <223> Primer
<400> 16
   aggatgctat ttagtctact taagct 26
<210> 17
   <211> 24
   <212> DNA
   <213> Artificial sequence
<220>
   <223> Primer
<400> 17
   ccagatgccg aataagtgta gtta 24
<210> 18
   <211> 24
   <212> DNA
   <213> Artificial sequence
<220>
   <223> Primer
<400> 18
   gcccttaaga tcagatacaa caga 24
<210> 19
   <211> 24
   <212> DNA
   <213> Artificial sequence
<220>
   <223> Primer
<400> 19
   aagactagaa ctccgtgagt gata 24
<210> 20
   <211> 24
   <212> DNA
   <213> Artificial sequence
<220>
   <223> Primer
<400> 20
   atacacccgt acgtaatcac aatc 24
<210> 21
   <211> 24
   <212> DNA
   <213> Artificial sequence
<220>
   <223> Primer
<400> 21
   gcaatttaat ccgcctactc atat 24
<210> 22
   <211> 24
   <212> DNA
   <213> Artificial sequence
<220>
   <223> Primer
<400> 22
   atcaaccgta cgtttgtatt tcac 24
<210> 23
   <211> 21
   <212> DNA
   <213> Artificial sequence
<220>
   <223> Primer
<400> 23
   cagcgttgcg aaacttatca t 21
<210> 24
   <211> 24
   <212> DNA
   <213> Artificial sequence
<220>
   <223> Primer
<400> 24
   gacttttcgc acacatatga gtag 24
<210> 25
   <211> 24
   <212> DNA
   <213> Artificial sequence
<220>
   <223> Primer
<400> 25
   cgtatttccg gtattctaat ccct 24
<210> 26
   <211> 24
   <212> DNA
   <213> Artificial sequence
<220>
   <223> Primer
<400> 26
   gcacgtattt ccggtattct aatc 24
<210> 27
   <211> 23
   <212> DNA
   <213> Artificial sequence
<220>
   <223> Primer
<400> 27
   atccgaggtt ctcacgaaat tag 23
<210> 28
   <211> 22
   <212> DNA
   <213> Artificial sequence
<220>
   <223> Primer
<400> 28
   ctcgcatcaa caagtcgtaa ag 22
<210> 29
   <211> 450
   <212> DNA
   <213> Artificial sequence
<220>
   <223> Template
<400> 29
   aaattcgctc tactccaacc acataatagt cctcacccaa acacactcgc cagttccgat 60
   actaaggctc tgccggcgct ctctttgaga tatcggttcg aaatcgacca cagtataaac 120
   ggatatagga acactgttag ttcgcatcca agcagtaggg gccagacctg tctcgctttg 180
   tacgatactc ggttagtaga ccaatgccaa ctaaggggat gtgttcacgg catttcggag 240
   ttcaaagggt tggtgagaag agctctcgaa gttgtttggg tgactcgatc ctaggaacct 300
   acggcttatc gatacaaacc cacagctttc cgtccatgtg aagtatggga tgcgcaagac 360
   ctgagcagcc gcgtgaggtc tacaaagttt aaaccgccgt tgtgtacggt agcgcatctt 420
   ctgattatgg agctacgcct atcgctcgct 450
<210> 30
   <211> 255
   <212> DNA
   <213> Artificial sequence
<220>
   <223> Template
<400> 30
   agccaactca gtcaatctca aaccgttttc cggtgttggt ggtgattacg tatcttagtc      60
   ccgctggccg ggtaatgata ccacgaatca attctcgaac tatgctgctt ccccgatccc     120
   gccccgcctt aagactttat gctcacgcgt tacggctcga aagatagtcc aggcgagacg     180
   tgtgtagagt tatgcgaaac gatctcagac gcgtttagcg gttaaaatgt cacacaaggg     240
   cattacccga ccggg                                                      255
<210> 31
   <211> 255
   <212> DNA
   <213> Artificial sequence
<220>
   <223> Template
<400> 31
   cgaacggggt ccggtatctg accgtcacat gtgccatcct cttggtcgta cgggctatcc      60
   actctagcaa acgacttact ttgacggtaa tggcaggccc ctaactacac ttattcggca     120
   tctgggtgtt tacgatgacg ctcctcccgc tatggtcaag cgacaggagt ggagatcatc     180
   gtacacgtcc caatgcatat attcacccgg ccacgtatcc gtgcaggttg ccgtgggtct     240
   gggagtgaac gtgcg                                                      255
<210> 32
   <211> 255
   <212> DNA
   <213> Artificial sequence
<220>
   <223> Template
<400> 32
   tcccgccctt aagatcagat acaacagaaa gcaagcaccc cgcctaggaa cagccccgtt      60
   gccgtcaatt actcttaatt ccaacggatt gttgctctcc ggagagaagg aaagtaagac     120
   tgtcgccggg ttacgtgtgt ttgtatccaa atcgccgaga gactcgatga ccattcggga     180
   gttgtaccca agaagccgcg ggtaaggcgt gcgggactca actcccaaat agtctcagtc     240
   cctcgaggta tatag                                                      255
<210> 33
   <211> 255
   <212> DNA
   <213> Artificial sequence
<220>
   <223> Template
<400> 33
   gatcaaccgt acgtttgtat ttcacactta cacttgcaat ttaatccgcc tactcatatg        60
   tgtgcgaaaa gtcggaggct agaactgtag cccgaatcgg ccgcaaggac ccactaaacg       120
   cgtagttgat atttcaaccg tagtagttgg gttcccactt aggccgagcc gagaggtcct       180
   ggatagtggt cagtagttaa gaggtgtctc ggtaagtata gagcaagtca cggccgaata       240
   catgccaact ccccc                                                        255
<210> 34
   <211> 255
   <212> DNA
   <213> Artificial sequence
<220>
   <223> Template
<400> 34
   gggcacgtat ttccggtatt ctaatccctg catgagaggg cctccacatc aacacgcgtc        60
   cagctttacg acttgttgat gcgagtgacc gtcgctatac tccgcttgcc cgttctgagg       120
   ggaactcacg tatcggccct tgataatgcg ttcgtcacag ggttcctaaa gtcgggcaca       180
   gtgagctgaa aggtgcatcc gtgccaaatg gaacctgtgg tggccacact cgggttaaac       240
   tcagcgctag tgaaa                                                        255

## Description

Molecular biology techniques are widely used in genotyping applications and other areas such as biological research, forensic and diagnostic applications, including human identification and paternity testing and for diagnosis of infectious diseases or chimera analysis after allogeneic bone marrow transplantation as well the detection of genetic diseases and cancer. The most commonly used technique is the polymerase chain reaction (PCR) that allows the researchers to amplify the desired DNA requiring only tiny amounts of sample.

Forensic workflow schemes entail the amplification of so called short tandem repeat (STR) markers. These markers are genetic elements of variable lengths that are characterized by short repetitive sequence motifs and are used in combination with other STR loci in order to obtain a genetic fingerprint of an individual. This information can be further used to accurately identify or eliminate a suspect by comparing this fingerprint with evidence from the crime scene.

After amplification, the resulting PCR products are labelled using fluorescent dyes and the technique of capillary electrophoresis (CE) is employed to separate said amplification products according to their molecular size. The fluorescent signals are represented as peaks in the electropherogram.

However, such analyses are hampered by several practical issues that prevent accurate genotyping. At the level of the template, possible degradation of the sample complicates efficient amplification of the target sequence. Further, naturally occurring compounds such as hematin or humic acid are potent inhibitors of PCR reactions. Since forensic samples are usually only available in very small quantities, the extent of possible trouble shooting in order to obtain a conclusive result is very limited.

Current means to provide an internal amplification control are hampered by non-specific binding that complicates subsequent analysis and do not allow for the distinction of the presence of inhibitors and template degradation WO2014/028531 relates to the development of a highly sensitive quantitative system for assessing DNA degradation and quality of forensic samples. Therefore, there is a need for a system that allows the researcher to differentially identify said problems in amplification and detection experiments.

### Brief description of the invention

The invention addresses the technical problems described above by means of an internal amplification control consisting of a set of primers and a template. The invention is defined in the appended claims. The present disclosure relates to a method for evaluating the amplification efficiency and/or the presence of inhibitors and/or degradation in an amplification reaction comprising a reaction mixture comprising
i. one or more internal nucleic acid templates with a length of between 50 and 2000 nucleotides,
   ii. at least 3 primers or 2 pairs of primers, which allow the generation of a first internal amplification product of between 20 and 800 nucleotides and at least one further internal amplification product of between 50 and 2000 nucleotides, wherein the at least 3 primers or 2 pairs of primers are specific for the one or more internal nucleic acid templates and the first and second internal amplification product are amplification products of the one or more internal nucleic acid templates, wherein the first internal amplification product is smaller than the second internal amplification product,
   iii. a sample nucleic acid to be analysed and primers specific for said nucleic acid and reagents for said amplification,
   wherein an amplification reaction is performed with said reaction mixture and the ratio of the smaller internal control amplification product to the larger internal control amplification product is analysed.

The present disclosure relates to a kit for evaluating the amplification efficiency and/or the presence of inhibitors and/or degradation in an amplification reaction, comprising
i. a nucleic acid to serve as an internal nucleic acid template and
ii. at least 3 primers that are specific for said internal nucleic acid template and wherein said primers generate a smaller and larger amplification product of the internal nucleic acid template and
iii. reagents for amplification and detection of nucleic acids.

### Detailed description of the invention

A "primer" herein refers to an oligonucleotide comprising a sequence that is complementary to a nucleic acid to be amplified or transcribed ("template"). During replication a polymerase attaches nucleotides to the 3'-OH end of the primer complementary to the respective nucleotides of the template.

The present disclosure relates to a method for evaluating the amplification efficiency and/or the presence of inhibitors and/or degradation in an amplification reaction comprising a reaction mixture comprising
i. one or more internal nucleic acid templates with a length of between 50 and 2000 nucleotides,
   ii. at least 3 primers or 2 pairs of primers, which allow the generation of a first internal amplification product of between 20 and 800 nucleotides and at least one further internal amplification product of between 50 and 2000 nucleotides, wherein the at least 3 primers or 2 pairs of primers are specific for the one or more internal nucleic acid templates and the first and second internal amplification product are amplification products of the one or more internal nucleic acid templates, wherein the first internal amplification product is smaller than the second internal amplification product,
   iii. a sample nucleic acid to be analysed and primers specific for said nucleic acid and reagents for said amplification,
   wherein an amplification reaction is performed with said reaction mixture and the ratio of the smaller internal control amplification product to the larger internal control amplification product is analysed.

In particular the method relates to the use of an internal control nucleic acid and its amplification as an internal control.

In one embodiment the internal nucleic acid template or the one or more internal nucleic acid templates are artificial nucleic acid templates. In an alternative embodiment the internal nucleic acid template or the one or more internal nucleic acid templates consist of a sequence of an organism, which is different than the origin or suspected origin of the sample nucleic acid to be analyzed. In a preferred embodiment the internal nucleic acid template or the one or more internal nucleic acid templates consist of a sequence with a very low identity to sequences in the sample. In a preferred embodiment the internal nucleic acid template or templates have been generated using a random algorithm. In a more preferred embodiment the internal nucleic acid template or templates comprise any of the non-human artificial sequences SEQ ID NOs 29 to 34. In a most preferred embodiment the internal nucleic acid template or templates consist of a sequence selected from the group comprising SEQ ID NOs 29, 30, 31, 32, 33 and 34.

In a preferred embodiment the internal nucleic acid template or templates have a length between 70 and 2000 nucleotides. In a preferred embodiment the internal nucleic acid template or templates have a length between 150 and 1500 nucleotides. In a more preferred embodiment the internal nucleic acid template or templates have a length between 150 and 1000 nucleotides. In an even more preferred embodiment the internal nucleic acid template or templates have a length between 200 and 500 nucleotides.

More preferably the internal nucleic acid template or templates have a length between 50 and 2000 nucleotides. In a preferred embodiment the internal nucleic acid template or templates have a length between 50 and 1500 nucleotides. In a more preferred embodiment the internal nucleic acid template or templates have a length between 50 and 1000 nucleotides. In an even more preferred embodiment the internal nucleic acid template or templates have a length between 50 and 500 nucleotides.

The method relates to at least two internal amplification products, wherein one amplification product has a shorter length than the other. These amplification products are referred to herein as first and second amplification product. Preferably the first amplification product has a length of at least 20 nucleotides and the second amplification product has a length of at least 70 nucleotides. The difference in length of the first and second amplification product has to be detectable.

The present disclosure further relates to primers or sets of primers to amplify the internal nucleic acid template or templates. If the internal nucleic acid template or templates comprise a sequence selected from SEQ ID NOs 29 to 34 it is preferred that the primers are selected from the group comprising SEQ ID NOs 1 to 28.

In one embodiment the present disclosure relates to a method for evaluating the amplification efficiency and/or the presence of inhibitors and/or degradation in an amplification reaction, wherein the analysis of the ratio of said first smaller internal control amplification product to said second larger internal control amplification product is performed by a method selected from the group comprising agarose gel electrophoresis, capillary electrophoresis, qPCR, digital PCR and next generation sequencing. The use of capillary electrophoresis is preferred, wherein the resulting amplification products are separated according to their molecular size and fluorescent dye label. The fluorescent dyes are frequently used as molecular labels attached to probes or other biomolecules.

The amplification may be a multiplex PCR. Multiplex PCR is for example used for the simultaneous detection of multiple marker genes and/or their polymorphisms, e.g. short tandem repeats (STRs) or deletion insertion polymorphisms (DIPS or Indels). Detection of the amplification products and their genotyping is usually carried out by multiple colour fluorescence detection after electrophoretic separation (e.g. capillary gel electrophoresis) in DNA sequencers. In real-time, quantitative multiplex PCR, the amplification of multiple target sequences can be monitored at the same time by simultaneous detection of fluorescence of different fluorescent dyes.

The amplification reaction is preferably selected from the group comprising polymerase chain reaction (PCR), ligase chain reaction (LCR), transcription-based amplification system (TAS), nucleic acid sequence based amplification (NASBA), rolling circle amplification (RCA), transcription-mediated amplification (TMA), self-sustaining sequence replication (3SR), QP amplification and (thermostable) helicase dependent amplification ((t)HAD). More preferably the amplification reaction is a PCR. Most preferably, the amplification reaction is a multiplex PCR, i.e. the amplification of more than one target nucleic acid sequence in a single tube, e.g. 2, 3, 4, 5, 6, 7, 8, 9, 10, 11, 12, 13, 14, 15,16, 17,18, 19, 20, 21, 22, 23, 24, 25, 26, 27, 28, 29, 30, 31, 32, 33, 34, 35, 36, 37, 38, 39 or 40 loci (target nucleic acid sequences) may be amplified simultaneously.

In another embodiment the disclosure relates to a method for evaluating the amplification efficiency and/or the presence of inhibitors and/or degradation in an amplification reaction, wherein the analysis of the ratio of said first smaller internal control amplification product to said second larger internal control amplification product is performed by a method called "Rapid DNA Analysis". Herein DNA from buccal and blood samples is processed by a fully automated DNA analysis system in about 90 minutes. Commercially available systems are for example RapidHIT™ (IntegenX), DNAscan (GE Healthcare Life Sciences, NetBio® Inc.). Rapid DNA Analysis Systems automatically extract DNA, perform the amplification of STR marker regions, separate the DNA fragments via capillary electrophoresis and produce standardized DNA profiles.

In a preferred embodiment the disclosure relates to a method for evaluating the amplification efficiency and/or the presence of inhibitors and/or degradation in an amplification reaction, wherein in addition several STR markers are amplified.

In a more preferred embodiment the disclosure relates to a method for evaluating the amplification efficiency and/or the presence of inhibitors and/or degradation in an amplification reaction, wherein the amplification reaction is performed in the context of genotyping. Such genotyping typically involves the amplification of a set of STR markers using multiplex PCR.

Analysis of the target amplification products and the internal control fragments allows for the differential identification of the presence of inhibitors or degradation of the sample.

The invention relates to a method for evaluating the amplification efficiency and/or the presence of inhibitors and/or degradation in an amplification reaction, wherein an equal ratio of the small and the larger internal control amplification products suggests a non-inhibited sample. Herein, an equal ratio of the small and the larger internal control amplification products together with a ratio in favour of small target products of the sample suggests a degraded sample. A shift of a ratio in favour of the small internal amplification product suggests the presence of one or several inhibitors in the sample. In the case that no target product is detected in the analysis of the amplification products, the presence of both the large and the small internal control amplification fragments suggests that the sample did not comprise amplifiable nucleic acids. The disclosure further relates to a nucleic acid, wherein the nucleic acid comprising a sequence elected from the group consisting of SEQ ID NO: 1 to 28 is used as a primer or a probe. These sequences are used in amplification reactions to generate said two internal amplification products.

The disclosure relates to the use of a nucleic acid according to SEQ ID NO 29 to 34 as an internal nucleic acid template in an amplification reaction. Said template is preferably used in a multiplex PCR reaction, wherein a set of STR markers are analysed. Said two internal control products of different sizes are amplified using that internal template.

The disclosure relates to a kit for evaluating the amplification efficiency and/or the presence of degradation and/or degradation in an amplification reaction, comprising
i. one or more nucleic acid to serve as an internal nucleic acid template and
ii. at least 3 primers or at least two pairs of primers that are specific for said one or more internal nucleic acid templates and wherein said primers generate a smaller and larger amplification product of the one or more internal nucleic acid templates and
iii. reagents for amplification and detection of nucleic acids.

### Examples

Previous STR analysis protocols entailed the use of primers and plasmids that originate from other organisms than the one of the sample to be analysed. In other cases, the same primers were used in both the internal control reaction and in the multiplex amplification of a target genomic region. However, this strategy of said parallel usage of primers is risky. In the case of forensic samples where the presence of PCR inhibitors and template quantity is critical, a drop in performance is observed in the amplification of the genomic target region. Using components from other kits as an internal control bears the risk of cross contamination.

Further, current methods did not provide a conclusive insight into whether an amplification reaction is hampered by the presence of a degraded template or by PCR inhibitors in said template.

### Design and bioinformatics analysis of template and primer sequences

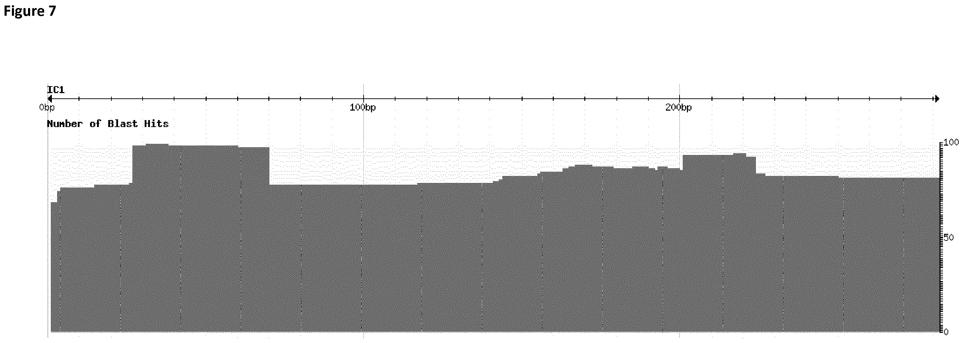

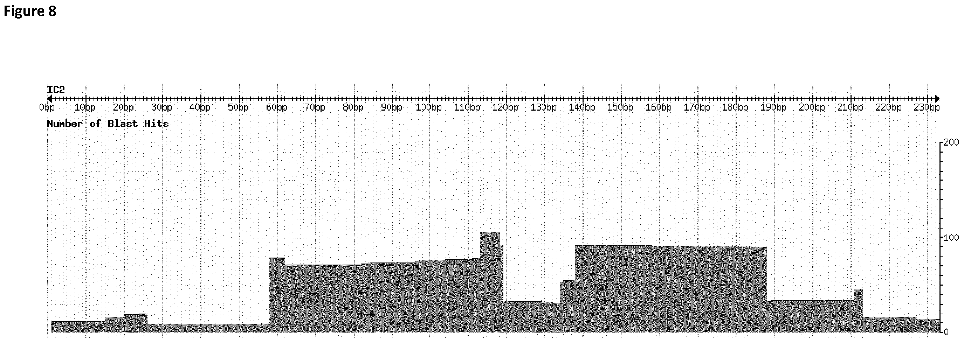

In order to assess sequence similarity of known internal control templates such as IC1 and IC2 to sequences available in public databases, BLAST searches were performed. As shown in Figure 7, in the case of IC1 at least 80 hits were identified. Some regions of the sequence show up to 100 hits. The BLAST search using IC2 also identified several hits across the sequence (Figure 8).

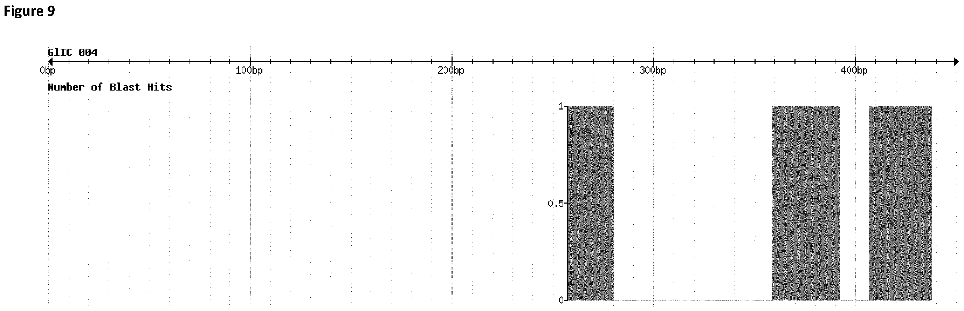

To address the aspect of sequence similarity and the possibility of non-specific binding synthetic DNA templates were designed using a random algorithm. A subsequent BLAST search yielded only up to 3 distinct hits spanning a short segment of 20 to 30 bp (Figure 9). The chance of non-specific binding in the context of a multiplex PCR amplification reaction is therefore very low. Primers (selected from SEQ ID NO 1 to 28) were designed based on these sequences to amplify two products of different sizes. The sequence of the primers and the first 30 nucleotides of the templates are shown in Table 1.

**Table 1: Primer and template sequences. The sequences of the primers and the templates are presented together with the corresponding SEQ ID NO and sequence annotation. Note that in the case of the templates (SEQ ID NO 29 to 34), only the first 30 nucleotides are presented. The complete sequences can be found in the sequence protocol.**

| **SEQ ID NO** | **Sequence annotation** | **Sequence** |
|---|---|---|
| 1 | GlIC_004_for1 | tactccaaccacataatagtcctc |
| 2 | GlIC_004_for2 | tctactccaaccacataatagtcc |
| 3 | GlIC_004_for3 | tcgctctactccaaccacataata |
| 4 | GlIC_004_for6 | cagccgcgtgaggtctacaaa |
| 5 | GlIC_004_for7 | attcgctctactccaaccacataatag |
| 6 | GlIC_004_rev1 | gctccataatcagaagatgcgctac |
| 7 | GlIC_004_rev2 | gcgtagctccataatcagaagatgc |
| 8 | GlIC_004_rev3 | gtaggcgtagctccataatcagaagat |
| 9 | GlIC_004_rev5 | gtagctccataatcagaagatgcg |
| 10 | GlIC_048_for1 | tggtggtgattacgtatcttagtc |
| 11 | GlIC_048_for2 | cctagcctaagttcagatgtaagg |
| 12 | GlIC_048_rev1 | tcatttctgctagtctaccttagc |
| 13 | GlIC_048_rev2 | tcatttctgctagtctaccttagc |
| 14 | GlIC_058_for1 | tcgtacgggctatccactctag |
| 15 | GlIC_058_for2 | ctatccactctagcaaacgactta |
| 16 | GlIC_058_rev1 | aggatgctatttagtctacttaagct |
| 17 | GlIC_058_rev2 | ccagatgccgaataagtgtagtta |
| 18 | GlIC_073_for1 | gcccttaagatcagatacaacaga |
| 19 | GlIC_073_for2 | aagactagaactccgtgagtgata |
| 20 | GlIC_073_rev1 | atacacccgtacgtaatcacaatc |
| 21 | GlIC_074_for1 | gcaatttaatccgcctactcatat |
| 22 | GlIC_074_for2 | atcaaccgtacgtttgtatttcac |
| 23 | GlIC_074_rev1 | cagcgttgcgaaacttatcat |
| 24 | GlIC_074_rev2 | Gacttttcgcacacatatgagtag |
| 25 | GlIC_082_for1 | cgtatttccggtattctaatccct |
| 26 | GlIC_082_for2 | gcacgtatttccggtattctaatc |
| 27 | GlIC_082_rev1 | atccgaggttctcacgaaattag |
| 28 | GlIC_082_rev2 | ctcgcatcaacaagtcgtaaag |
| 29 | GlIC_004 | aaattcgctctactccaaccacataatagt |
| 30 | GlIC_048 | agccaactcagtcaatctcaaaccgttttc |
| 31 | GlIC_058 | cgaacggggtccggtatctgaccgtcacat |
| 32 | GlIC_073 | tcccgcccttaagatcagatacaacagaaa |
| 33 | GlIC_074 | gatcaaccgtacgtttgtatttcacactta |
| 34 | GlIC_082 | gggcacgtatttccggtattctaatccctg |

Analysis of the presence of these two internal template amplification products allows the researchers to differentially identify the presence of PCR inhibitors or the level of degradation in the forensic sample. When inhibitors such as hematin and humic acid are present in a sample, amplification is less efficient and especially larger DNA fragments are amplified less than smaller ones. In the case of sample degradation, smaller fragments are more abundant and larger target fragments are also amplified less efficiently. However, said degradation of the target template does not hamper amplification of the internal control fragments from the internal control template.

### Validation of internal amplification control

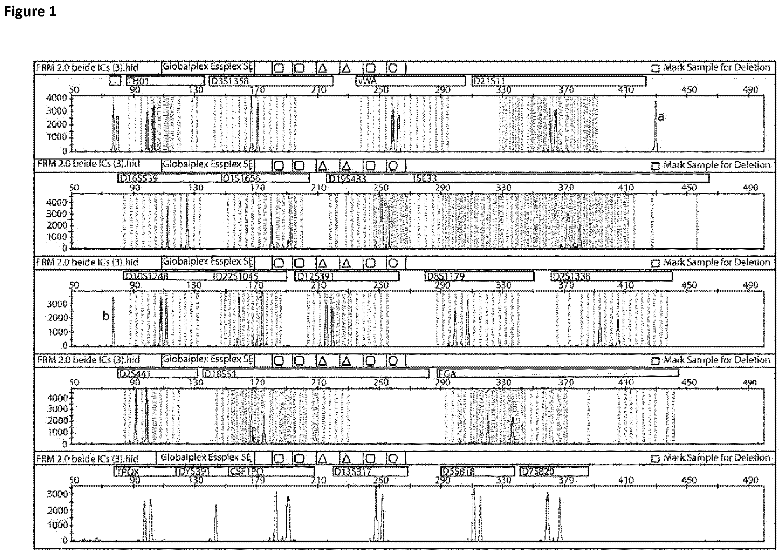

In order to validate said internal amplification template and primers in amplification experiments, STR analyses were performed. In the preliminary experiment, a set of 23 markers was amplified. As shown in Figure 1, all markers were amplified correctly and are represented in the electropherogram according to their molecular size and dye label. Further, both the large and the small internal control fragments were amplified correctly and can be identified in the electropherogram at the expected locations (Figure 1, a: large fragment, b: small fragment). This shows that the amplification reaction was successful and that the presence of the internal template and primers did not affect amplification of the target STR markers.

### Presence of inhibitors

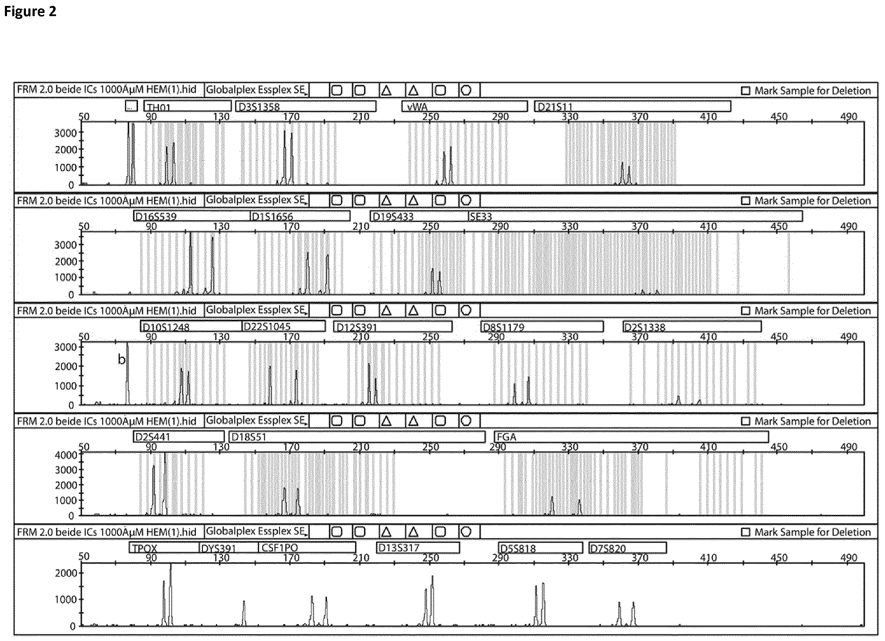

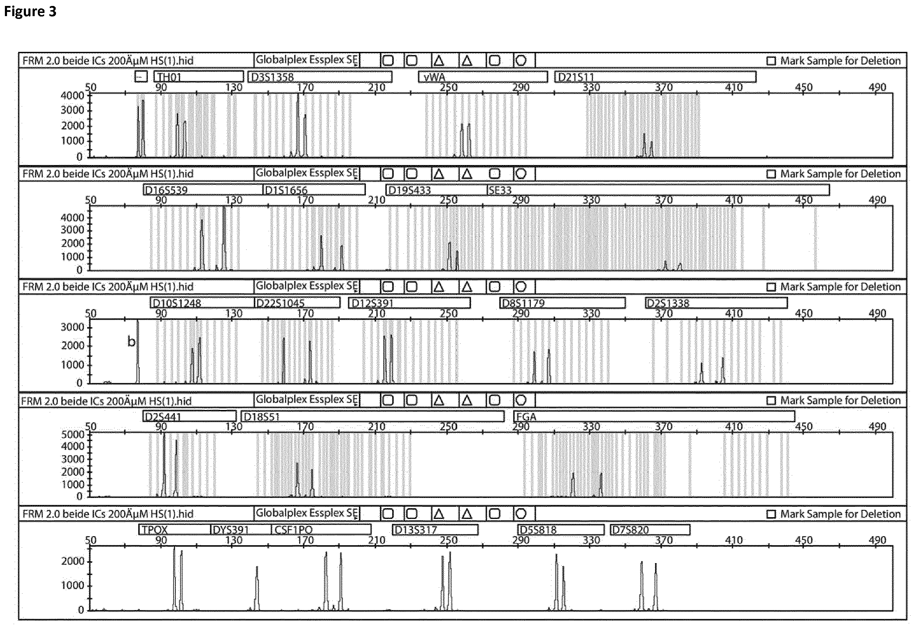

The subsequent experiments consisted of STR analyses in the presence of 1 mM hematin and 200 ng/µl humic acid. The presence of these compounds led to inefficient amplification of larger DNA fragments. The corresponding peaks in the electropherogram are either very small or not visible. Similarly, the larger internal control fragment was also not amplified efficiently. The small internal control fragment however was amplified and can be identified in the electropherograms (Figure 2 and 3, fragment b).

### Sample degradation

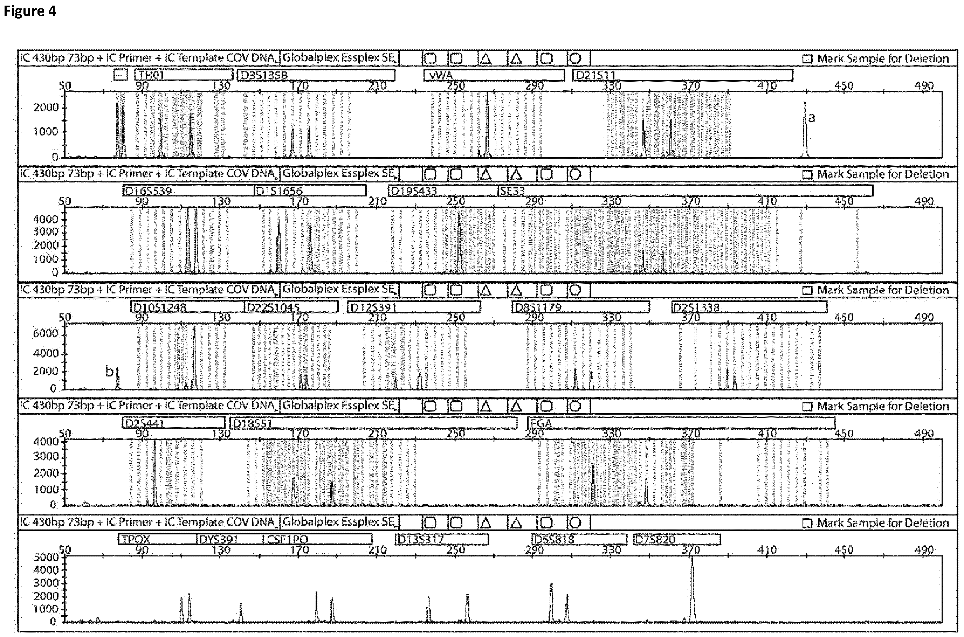

Further, the internal control primers and template were evaluated with regards to sample degradation. The preliminary experiment using intact genomic DNA showed that all STR markers were amplified and are represented in the electropherogram according to their molecular size and dye label. Both the large and the small internal control fragment were amplified (Figure 4, a and b).

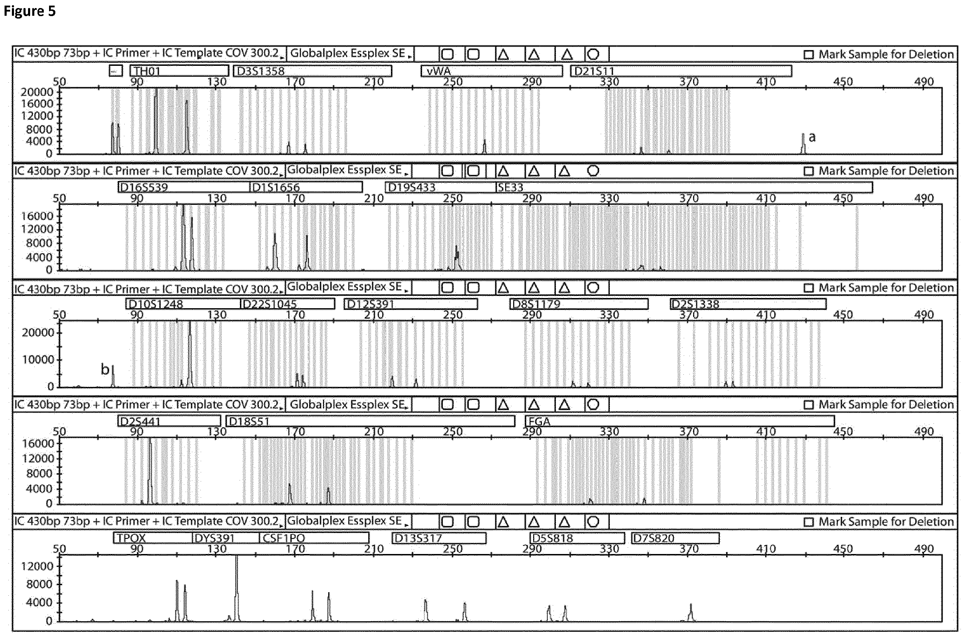

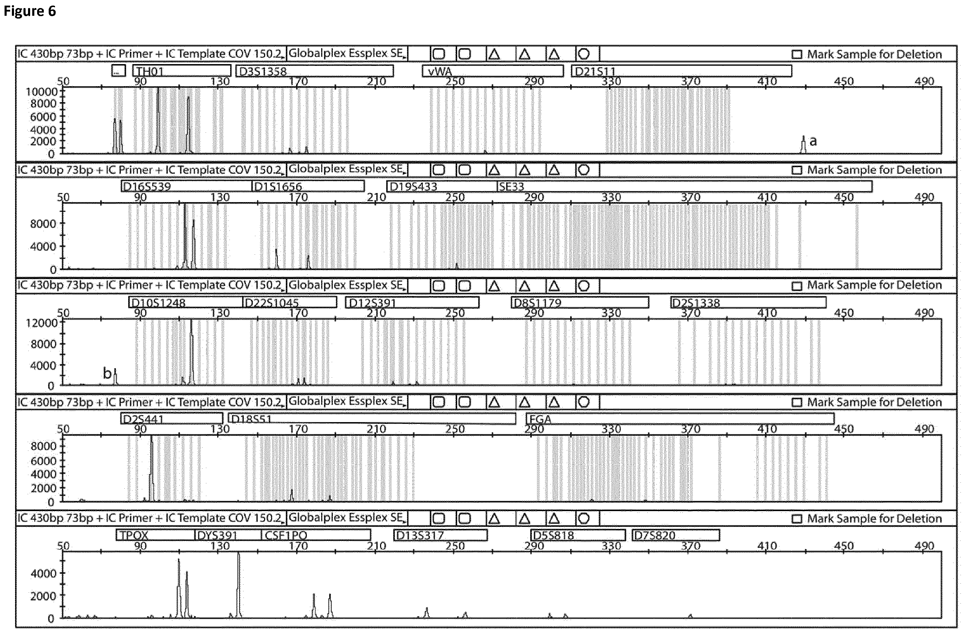

For the subsequent experiments said genomic DNA was sheared to fragments of 300 and 150 bp respectively using an ultra-sonic device (Covaris) and amplification reactions were performed. As shown in Figure 5 and 6, larger STR target fragments were not amplified efficiently and the corresponding peaks are either small or not present in the electropherograms. However, in both cases the large and the small internal control fragments were amplified irrespective of said degradation of the target template (Figure 5 and 6, a and b).

In summary, the examples show that a system comprising an internal artificial template together with three primers that amplify two fragments of distinct sizes from the internal control is a means to distinguish the presence of inhibitors from the DNA degradation level and to thereby evaluate amplification efficiency. Presence of inhibitors is characterized by inefficient amplification of both the larger target fragments and the larger internal control fragment whilst maintaining efficient amplification of the smaller internal control fragment. Degradation however does not affect efficient amplification of both internal control fragments.

### Figure captions

**Figure 1**
   **Electropherogram of STR analysis.** 23 STR markers were amplified and analysed using CE. The large and the small internal control fragments were amplified in the same reaction. Annotations are as follows: The respective markers are presented at the top of the electropherogram. a and b denote the large and the small internal control fragment respectively.
**Figure 2**
   **Electropherogram of STR analysis in the presence of hematin.** 23 STR markers were amplified in the presence of 1 mM hematin and analysed using CE. The large and the small internal control fragments were amplified in the same reaction. Annotations are as follows: The respective markers are presented at the top of the electropherogram. a and b denote the large and the small internal control fragment respectively. A peak corresponding to fragment a is absent.
**Figure 3**
   **Electropherogram of STR analysis in the presence of humic acid.** 23 STR markers were amplified in the presence of 200 ng/µl humic acid and analyzed using CE. The large and the small internal control fragments were amplified in the same reaction. Annotations are as follows: The respective markers are presented at the top of the electropherogram. a and b denote the large and the small internal control fragment respectively. A peak corresponding to fragment a is absent.
**Figure 4**
   **Electropherogram of STR analysis.** Genomic DNA was used in this experiment. Annotations are as follows: The respective markers are presented at the top of the electropherogram. a and b denote the large and the small internal control fragment respectively.
**Figure 5**
   **Electropherogram of STR analysis in the presence of DNA degradation (fragments of 300 bp).** Genomic DNA was used in this experiment and sheared to fragments of 300 bp using ultrasound. Annotations are as follows: The respective markers are presented at the top of the electropherogram. a and b denote the large and the small internal control fragment respectively.
**Figure 6**
   **Electropherogram of STR analysis in the presence of DNA degradation (fragments of 150 bp).** Genomic DNA was used in this experiment and sheared to fragments of 150 bp using ultrasound. Annotations are as follows: The respective markers are presented at the top of the electropherogram. a and b denote the large and the small internal control fragment respectively.
**Figure 7**
   **BLAST search using internal control IC1.** The sequence of IC1 is shown at the top. The number of BLAST hits is shown on the left. Grey areas represent the regions of the sequence per base that show hits in the BLAST search.
**Figure 8**
   **BLAST search using internal control IC2.** The sequence of IC2 is shown at the top. The number of BLAST hits is shown on the left. Grey areas represent the regions of the sequence per base that show hits in the BLAST search.
**Figure 9**
   **BLAST search using artificial template GIIC 004 (SEQ ID NO 29).** The sequence of GIIC 004 is shown at the top. The number of BLAST hits is shown on the left. Grey areas represent the regions of the sequence per base that show hits in the BLAST search.

## Claims

1. Method for evaluating the amplification efficiency and/or the presence of inhibitors and/or degradation in an amplification reaction comprising a reaction mixture comprising
i. one or more internal control nucleic acid templates with a length of between 50 and 2000 nucleotides,
ii. at least 3 primers or at least 2 pairs of primers, which allow the generation of a first internal control amplification product of between 20 and 800 nucleotides and at least one further internal control amplification product of between 70 and 2000 nucleotides, wherein the 3 primers or 2 pairs of primers are specific for the one or more internal control nucleic acid template and the first and second internal control amplification product are amplification products of the one or more internal control nucleic acid template, wherein the first internal control amplification product is smaller than the second internal control amplification product,
iii. a sample nucleic acid to be analysed and primers specific for a target nucleic acid and reagents for said amplification,
wherein the one or more internal control nucleic acid templates are artificial nucleic acid templates or consist of a sequence of an organism, which is different than the origin or suspected origin of the sample nucleic acid to be analyzed, and
wherein an amplification reaction is performed with said reaction mixture and the ratio of the smaller internal control amplification product to the larger internal control amplification product is analysed, and
wherein an equal ratio of the small and the larger internal control amplification products suggests a non-inhibited sample,
an equal ratio of the small and the larger internal control amplification products together with a ratio in favour of small target products of the sample suggests a degraded sample, a shift of a ratio in favour of the small internal control amplification product suggests the presence of one or several inhibitors in the sample and
if no target product is detected in the analysis of the amplification products, the presence of both the large and the small internal control amplification fragments suggests that the sample did not comprise amplifiable nucleic acids.

2. Method for evaluating the amplification efficiency and/or the presence of inhibitors and/or degradation in an amplification reaction according to claim 1, wherein the analysis of the ratio of said first smaller internal control amplification product to said second larger internal control amplification product is performed by a method selected from the group comprising agarose gel electrophoresis, capillary electrophoresis, qPCR, digital PCR and next generation sequencing.

3. Method for evaluating the amplification efficiency and/or the presence of inhibitors and/or degradation in an amplification reaction according to claim 1 or 2, wherein in addition several STR markers are amplified.

4. Method for evaluating the amplification efficiency and/or the presence of inhibitors and/or degradation in an amplification reaction according to claims 1 to 3, wherein the amplification reaction is performed in the context of genotyping.

5. Kit for evaluating the amplification efficiency and/or the presence of inhibitors and/or degradation in an amplification reaction, comprising
i. one or more nucleic acids selected from the group of SEQ ID NO 29 - 34 to serve as an internal control nucleic acid template and
ii. at least 3 primers or at least two pairs of primers that are specific for said internal control nucleic acid templates and wherein said primers generate a smaller and larger control amplification product of the internal control nucleic acid template and
iii. reagents for amplification and detection of nucleic acids.

## Patentansprüche

1. Verfahren zur Bewertung der Amplifikationseffizienz und/oder der Anwesenheit von Inhibitoren und/oder Degradation in einer Amplifikationsreaktion umfassend eine Reaktionsmischung umfassend
i. eine oder mehrere interner Kontrolle Nukleinsäuretemplate mit einer Länge zwischen 50 und 2000 Nukleotide,
ii. mindestens 3 Primer oder mindestens 2 Primerpaare, die die Erzeugung eines ersten internen Kontroll-Amplifikationsprodukts ermöglichen von zwischen 20 und 800 Nukleotiden und mindestens ein weiteres internes Kontroll-Amplifikationsprodukt zwischen 70 und 2000 Nukleotide, wobei die 3 Primer oder 2 Primerpaare spezifisch für das eine oder die mehreren internen Kontroll-Nukleinsäuretemplates sind und das erste und das zweite interne Kontroll-Amplifikationsprodukt Amplifikationsprodukte des einen oder der mehreren internen Kontroll-Nukleinsäuretemplats sind, wobei die erste interne Kontroll-Amplifikation ist Produkt kleiner als das zweite interne Kontroll-Amplifikationsprodukt ist,
iii. eine zu analysierende Nukleinsäureprobe und für eine Zielnukleinsäure spezifische Primer und Reagenzien für diese Amplifikation,
wobei das eine oder die mehreren internen Kontroll-Nukleinsäuretemplates künstliche Nukleinsäuretemplates sind oder aus einer Sequenz eines Organismus bestehen, die sich von dem Ursprung oder vermuteten Ursprung der zu analysierenden Nukleinsäureprobe unterscheidet, und
wobei eine Amplifikationsreaktion mit der Reaktionsmischung durchgeführt wird und das Verhältnis des kleineren internen Kontroll-Amplifikationsprodukts zum größeren internen Kontroll-Amplifikationsprodukt analysiert wird, und wobei ein gleiches Verhältnis der kleinen und der größeren internen Kontroll-Amplifikationsprodukte eine nicht inhibierte Probe nahelegt,
ein gleiches Verhältnis der kleinen und der größeren internen Kontroll-Amplifikationsprodukte mit einem Verhältnis zugunsten kleiner Zielprodukte der Probe auf eine degradierte Probe hindeutet, eine Verschiebung eines Verhältnisses zugunsten des kleinen internen Kontroll-Amplifikationsprodukts auf das Vorhandensein eines oder mehrerer Inhibitoren in der Probe hinweist und
wenn bei der Analyse der Amplifikationsprodukte kein Zielprodukt nachgewiesen wird, das Vorhandensein sowohl der großen als auch der kleinen internen Kontroll-Amplifikationsfragmente nahelegt, dass die Probe keine amplifizierbaren Nukleinsäuren umfasst.

2. Verfahren zur Bewertung der Amplifikationseffizienz und/oder der Anwesenheit von Inhibitoren und/oder der Degradation in einer Amplifikationsreaktion gemäß Anspruch 1, wobei die Analyse des Verhältnisses des ersten kleineren internen Kontroll-Amplifikationsprodukts zu dem zweiten größeren internen Kontroll-Amplifikationsprodukt durch ein Verfahren ausgewählt aus der Gruppe umfassend Agarosegelelektrophorese, Kapillarelektrophorese, qPCR, digitale PCR und Next Generation Sequencing durchgeführt wird.

3. Verfahren zur Bewertung der Amplifikationseffizienz und/oder der Anwesenheit von Inhibitoren und/oder Degradation in einer Amplifikationsreaktion gemäß Anspruch 1 oder 2, wobei zusätzlich mehrere STR-Marker amplifiziert werden.

4. Verfahren zur Bewertung der Amplifikationseffizienz und/oder der Anwesenheit von Inhibitoren und/oder Degradation in einer Amplifikationsreaktion gemäß den Ansprüchen 1 bis 3, wobei die Amplifikationsreaktion im Rahmen der Genotypisierung durchgeführt wird.

5. Kit zur Bewertung der Amplifikationseffizienz und/oder der Anwesenheit von Inhibitoren und/oder Degradation in einer Amplifikationsreaktion, umfassend
i. eine oder mehrere Nukleinsäuren, ausgewählt aus der Gruppe der SEQ ID NO 29- 34, dienend als ein interner Kontroll-Nukleinsäuretemplate und
ii. mindestens 3 Primer oder mindestens zwei Primerpaare, die spezifisch für diesen internen Kontroll-Nukleinsäuretemplate sind und wobei die Primer ein kleineres und ein größeres Kontroll-Amplifikationsprodukt des internen Kontroll-Nukleinsäuretemplates erzeugen und
iii. Reagenzien für die Amplifikation und Nachweis von Nukleinsäuren.

## Revendications

1. Méthode pour évaluer l'efficacité d'amplification et/ou de la présence d'inhibiteurs et/ou de dégradation dans une réaction d'amplification comprenant un mélange de réaction comprenant
i. un ou plusieurs matrices d'acides nucléiques de contrôle interne d'une longueur compris entre 50 et 2000 nucléotides,
ii. au moins 3 amorces ou au moins 2 paires d'amorces, permettant la génération d'un premier produit d'amplification de contrôle interne compris entre 20 et 800 nucléotides et au moins un autre produit d'amplification de contrôle interne compris entre 70 et 2000 nucléotides, dans lequel les 3 amorces ou 2 paires d'amorces sont spécifiques pour l'un ou plus de les matrices d'acide nucléique de contrôle interne et les premier et second produits d'amplification de contrôle interne sont des produits d'amplification de l'un ou plus de les matrices d'acide nucléique de contrôle interne, dans lequel le premier produit d'amplification de contrôle interne étant plus petit que le deuxième produit d'amplification du contrôle interne,
iii. un échantillon d'acide nucléique à analyser et des amorces spécifiques pour un acide nucléique cible et des réactifs pour ladite amplification,
dans lequel l'un ou le plus des matrices d'acide nucléique de contrôle interne sont des matrices d'acide nucléique artificielles ou consistent en une séquence d'un organisme différente de l'origine ou l'origine présumée de l'acide nucléique de l'échantillon à analyser, et
dans lequel une réaction d'amplification est effectuée avec ledit mélange de réaction et le rapport du produit d'amplification de contrôle interne plus petit au produit d'amplification de contrôle interne plus grand est analysé, et
dans lequel un rapport égal des petits et des plus grands produits d'amplification de contrôle interne suggère un échantillon non-inhibé,
un rapport égal des petits et des plus grands produits d'amplification de contrôle interne avec un rapport en faveur de petits produits cibles de l'échantillon suggère une dégradation échantillon, un décalage d'un rapport en faveur du petit produit d'amplification du contrôle interne suggère la présence d'un ou plusieurs inhibiteurs dans l'échantillon et
si aucun produit cible n'est détecté dans l'analyse des produits d'amplification, la présence à la fois des fragments d'amplification grands et petits de contrôle interne suggère que l'échantillon ne comprend pas d'acides nucléiques amplifiables.

2. Méthode pour évaluer l'efficacité d'amplification et/ou de la présence d'inhibiteurs et/ou de dégradation dans une réaction d'amplification selon la revendication 1, dans lequel l'analyse du rapport de ledit premier produit d'amplification plus petit de contrôle interne audit second produit d'amplification plus grand de contrôle interne est réalisée par une méthode choisi dans le groupe comprenant l'électrophorèse sur gel d'agarose, l'électrophorèse capillaire, la qPCR, la *digital* PCR et le séquençage de nouvelle génération.

3. Méthode pour évaluer l'efficacité d'amplification et/ou de la présence d'inhibiteurs et/ou de dégradation dans une réaction d'amplification selon la revendication 1 ou 2, dans lequel plusieurs marqueurs STR sont en outre amplifiés.

4. Méthode pour évaluer l'efficacité d'amplification et/ou de la présence d'inhibiteurs et/ou de dégradation dans une réaction d'amplification selon les revendications 1 à 3, dans lequel la réaction d'amplification est effectuée dans le contexte du génotypage.

5. Kit pour évaluer l'efficacité d'amplification et/ou la présence d'inhibiteurs et / ou de dégradation dans une réaction d'amplification, comprenant
i. un ou plusieurs acides nucléiques choisis dans le groupe de SEQ ID N ° 29-34 pour servir de matrice d'acide nucléique de contrôle interne et
ii. au moins 3 amorces ou au moins deux paires d'amorces spécifiques de lesdites matrices d'acides nucléiques de contrôle interne et dans lequel lesdites amorces génèrent un produit d'amplification plus petit et plus grand de la matrice d'acide nucléique de contrôle interne et
iii. réactifs pour l'amplification et la détection des acides nucléiques.
